# EUROPEAN PATENT SPECIFICATION

(11) **EP 2 057 268 B1**
(45) Date of publication and mention of the grant of the patent: **13.08.2014**
(21) Application number: 06783600.7
(22) Date of filing: 14.08.2006
(51) Int. Cl.: C12N 15/51, A61K 39/39, A61K 48/00, A61P 1/16, A61P 31/12

(54) **A DNA VACCINE FOR CURING CHRONIC HEPATITIS B AND A METHOD OF PREPARING SAME**
DNA-IMPFSTOFF ZUR HEILUNG VON CHRONISCHER HEPATITIS B UND VERFAHREN ZUR HERSTELLUNG DAVON
VACCIN À ADN SERVANT À TRAITER L'HÉPATITE B CHRONIQUE, ET SON PROCÉDÉ DE PRÉPARATION

(43) Date of publication of application: 13.05.2009
(73) Proprietor: Postech Foundation, Gyeongsangbuk-do 790-784 (KR); Dong-A Pharmaceutical Co., Ltd., Seoul 130-072 (KR); Genexine, Inc., Bundang-gu, Seongnam-si Gyeonggi-do 463-400 (KR); DAEWOONG PHARMACEUTICAL CO., LTD., Sungnam-si Kyunggi-do 462-120 (KR)
(72) Inventor: SUNG, Young Chul, Pohang-si, Gyeongsangbuk-do 790-310 (KR); YANG, Se hwan, Pohang-si, Gyeongsangbuk-do 790-751 (KR); IM, Se jin, Pohang-si, Gyeongsangbuk-do 790-784 (KR); LEE, Chang geun, Daegu 701-770 (KR); PARK, Su hyung, Seoul 134-786 (KR); SONG, Man ki, Seoul 120-191 (KR); SON, Jong moon, Incheon 403-011 (KR); YOON, Seung kew, Seoul 137-040 (KR); KIM, Chae young, Suwon-si, Gyeonggi-do 440-709 (KR); KIM, Byung moon, Seoul 135-837 (KR); LEE, Sung hee, Seoul 151-795 (KR); KIM, Won bae, Seoul 135-971 (KR)
(74) Representative: Bassil, Nicholas Charles
(86) International application number: PCT/KR2006/003181
(87) International publication number: WO 2008/020656

(56) References cited:
- WO-A1-2005/042733
- WO-A1-2006/039739
- WO-A2-2006/002079
- LIU F ET AL: "Independent but not synergistic enhancement to the immunogenicity of DNA vaccine expressing HIV-1 gp120 glycoprotein by codon optimization and C3d fusion in a mouse model", VACCINE, ELSEVIER LTD, GB, vol. 22, no. 13-14, 16 April 2004 (2004-04-16), pages 1764-1772, XP004500431, ISSN: 0264-410X, DOI: DOI:10.1016/J.VACCINE.2003.09.054
- APT D ET AL: "Tetravalent neutralizing antibody response against four dengue serotypes by a single chimeric dengue envelope antigen", VACCINE, ELSEVIER LTD, GB, vol. 24, no. 3, 16 January 2006 (2006-01-16), pages 335-344, XP025151079, ISSN: 0264-410X, DOI: DOI:10.1016/J.VACCINE.2005.07.100 [retrieved on 2006-01-16]
- MOHAMED TAREK M. SHATA ET AL.: 'Attemped therapeutic immunization in a chimpanzee chronic HBV carrier with a high viral load' J. MED. PRIMATOL. vol. 35, no. 5, June 2006, pages 165 - 171, XP008104084
- FISSOLO N. ET AL.: 'DNA vaccines prime CD8+ T cell responses to epitopes of viral antigens produced from overlapping reading frames of a single coding sequence' EUR. J. IMMUNOL. vol. 35, 2005, pages 117 - 127, XP008104068
- FRELIN L. ET AL.: 'Codon optimization and mRNA amplification effectively enhances the immunologenicity of the hepatitis C virus nonstructural 3/4A gene' GENE THER. vol. 11, no. 6, 2004, pages 522 - 533, XP002285893
- QIN S. ET AL.: 'Construction of recombinant plasmids containing genes of HBsAg and their expression in the eukaryotic cells' HUA XI. YI. KE. DA. XUE. XUE. BAO vol. 32, no. 2, 2001, pages 172 - 174, XP008104107

## Description

### [Technical Field]

The present invention relates to a DNA vaccine for curing chronic Hepatitis B virus, and a method of preparing the DNA vaccine.

### [Background Art]

Since the first case report of HBV (Hepatitis B virus) in 1966, HBV is now known to have infected more than 300 million people worldwide and approximately 50 million people are newly infected with HBV. Chronic hepatitis B infection accounts for approximately 80% of liver cancer patients worldwide and approximately 75% of HBV cases are found in Asian countries. In addition, HBV infections lead one in every four persons to severe liver diseases, including liver cancer and death. HBV affects approximately 5-8% of the Korea's population.

Transmission from mother to infant during the perinatal period is a major source of HBV transmission in Korea. Particularly, approximately 90% of infants who become infected will develop chronic HBV infection.

Currently clinically approved drugs for use in treatment of chronic hepatitis B include lamivudine, and interferon-alpha (IFN-α). Recently, adefovir and entecavir have been approved by the Food and Drug Administration (FDA) for use in treatment of chronic HBV infection. Lamivudine (Brand name: Fpivir-HBV; Manufacturer: GlaxoSmithKline) is a nucleoside analog and primarily serves to inhibit the activity of an HBV polymerase: Lamivudine effectively suppresses virus replication during a treatment period. However, immediately after cessation of treatment, viral rebound occurs in the majority of patients, resulting in liver injury. The treatment efficacy of lamivudine is temporary and is as low as about 20%. In addition, long-term efficacy of lamivudine has been limited due to emergency of YMDD-mutants of HBV during lamivudine treatment, that is, approximately 24% after one year of treatment, approximately 38% after two years of treatment, approximately 54% after three years of treatment, and approximately 66% after four years of treatment, as described in an article by Leung NW, et al, Hepatology 2001, 33:1527-1532. Adefovir and entecavir also act as competitive inhibitors of HBV polymerase. While adefovir and entecavir demonstrate substantially the same efficacy level as lamivudine, they have much smaller incidence rates of drug-resistant HBV mutants than lamivudine, allowing improved therapy over lamivudine in view of drug-resistance (NEJM 2003, 348:800; NEJM 2003, 348:808). However, these chemical drugs had fundamental limitation in which serum HBV DNA levels and alanine aminotransferase (ALT) levels may increase again in most patients after withdrawal of administration. IFN- a(Brand name: Intron-A; Manufacturer: Schering-Plough) generally increases the immune response of a host, whereby exhibits therapeutic effects such as inhibiting reproduction of HBV. However, IFN-α may cause serious side effects and therapeutic effect of IFN-α is merely 25 - 30%. Accordingly, in order to overcome the shortcomings of the existing therapeutic drugs, there is a need for new therapeutic drugs with high safety and efficacy.

Many recent clinical studies have emphasized the importance of a CD4 T cell immune response, as described in Liver, 1998, 18:405; Vaccine. 2001, 19: 2395, or a CD8 T cell immune response, as described in J Exp Med. 1995, 181:1047; J Infect Dis. 1993, 168:1133, which are more potent, specific to multiple antigens (to be abbreviated to "multi-specific" hereinafter) for the treatment and recovery of chronic hepatitis B patients. That is to say, patients naturally having recovered from hepatitis B showed multi-specific and sustained CD4 and CD8 T cell immune responses, while chronic hepatitis B patients showed weak and temporary CD4 and CD8 T cell immune responses, which are induced only to some HBV epitopes. In addition, there have been reports that these T cells have poor proliferation capacity and low cytokine synthesis capability. Approximately 1-2% of chronic hepatitis B patients have found to be naturally cured. Before seroconversion of HBsAg, which is one of treatment markers for chronic hepatitis B, a cell-mediated immune response is observed, by which the cell-mediated immune response is important in the treatment of chronic hepatitis B (J Hepatol, 2006 2; [Epub ahead of print]). These researches support demands for therapeutics capable of inducing a potent and multi-specific, cell-mediated immune response for the treatment of hepatitis B.

The first attempt made for the treatment of hepatitis 13 was recombinant protein vaccine using surface antigen. After initial 6-month treatment with the recombinant protein vaccine, a vaccine-treated group seemed cured in terms of loss of HBV DNA and induction of HBsAg seroconversion. However, after 1-year treatment with the recombinant protein vaccine, no difference in the efficacy of the recombinant protein vaccine was made between vaccine-treated group and placebo-controlled group (J Hepatol, 2001 34; 917). Even when a HBV large protein was used as the vaccine, neither CD4 T cell response nor antigen-specific CD8 T cell response was induced (Vaccine, 2002 20; 3598). These results indicate that while the recombinant protein vaccine can be effective in preventing HBV infection, it would not be suitable for the treatment of hepatitis B. An alternative attempt was made to induce a CD8 T cell response using peptide epitope specifically recognized by cytotoxic T lymphocytes (CTL). The peptides of HBV core-specific helper T-cell (Th) or CTL epitopes and tetanus toxoid (TT) as a control were administered to normal individuals, and a CTL response specific to the antigen was well recognized (J Clin Invest, 1995 95;341). However, the test with chronic HBV patients showed weak CTL response, and association with viral reduction was detectable (Hepatology, 1999 30: 531).

Another attempt for treating chronic hepatitis B is DNA vaccination for encoding HBV antigens. A DNA vaccine is advantageous in that an antigen can be expressed in the DNA in vivo, undergoing both endogenous and exogenous processes to be loaded on MHC class I and II, thereby simultaneously inducing CD4 and CD8 T cell responses. In practice, when DNA vaccines encoding envelope antigen were administered to normal individuals, both an antibody response and a cell-mediated immune response were induced, and no particular side effects were detected. However, when DNA vaccines encoding envelope protein (HBV PreS2-S) were administered to patients with chronic hepatitis B, HBV DNA loss was observed from only one of 10 patients (Hepatology, 2004 40: 874). In addition, T cell proliferation capability was induced to two patients, but the induced T cell responses were only transient and very weak. Adcordingly, while immune responses were induced to several patients, there still exists a need for improvement of DNA vaccines which can induce cell-mediated immune responses in a more potent and effective manner.

WO20040361210 A2 describes the use of a multiple-epitope construct for inducing an immune response against HBV in an individual. DE10339927 A1 describes the use of at least two surface antigens of HBV and by excluding the use of the HBV core antigen. US6249201 B1 describes a nucleic acid vaccine composed of surface antigens of HBV.

### [Disclosure]

### [Technical Solution]

The present invention provides a new DNA vaccine for treatment of chronic Hepatitis B, and a method for preparing the DNA vaccine. The subject matter for which protection is sought is as defined in the claims.

In particular the present invention provides a Hepatitis B virus (HBV DNA vaccine <11> comprising two expression vectors, wherein one expression vector comprises polynucleotide sequences encoding hepatitis B surface antigen (HBsAg) and L protein, and the other expression vector comprises polynucleotide sequences encoding core protein and polymerase, wherein the polynucleotide sequence encoding HBsAg comprises the polynucleotide sequence of SEQ. ID. NO: 1, the polynucleotide sequence encoding L protein comprises the polynucleotide sequence of SEQ. ID. NO: 2, the polynucleotide sequence encoding core protein comprises the polynucleotide sequence of SEQ. ID. NO: 9, and the polynucleotide sequence encoding polymerase comprises the polynucleotide sequence of SEQ. ID. NO: 10.

In the present invention, the DNA vaccine may further include IL-12, furthermore IL-12 variant, as an immune adjuvant.

The present invention further provides a method for providing the DNA vaccine.

The present invention also provides the use of the DNA vaccine in a method for curing

### chronic Hepatitis B.

### [Description of Drawings]

FIG. 1 is a schematic diagram of HB-110, which is a DNA vaccine according to a preferred embodiment of the present invention. HB-110 consists of three expression vectors; pGX10 S/L constructed to express HBV surface antigen (HBsAg) and L protein, pGX10 core/Pol constructed to express HBV core and polymerase, and pGX10 IL-12m constructed to express interleukin 12 variant.
FIG. 2 illustrates gene expression enhanced by codon-optimizing the gene of HBsAg (FIG. 2A) and the gene of HBV core (FIG. 2B).
FIG. 3 is a graphical representation of HBsAg levels expressed in C2C12 and HeLa cells by HB-100 and HB-110 DNA vaccines as determined by ELISA.
FIG. 4 is a graphical representation for comparison of HBV L glycoprotein levels expressed in COS-7 and HeLa cells by HB-100 and HB-110 DNA vaccines as determined by Western blot and ELISA.
FIG. 5 is a graphical representation for comparison of HBV core antigen levels expressed in COS-7 cells by HBV-100 and HB-110 DNA vaccines as determined by radioimmunoprecipitation (RIP).
FIG. 6 is a graphical representation of HBV polymerase antigen levels expressed in COS-7 cells by HB-110 DNA vaccine as determined by Western blot.
FIG. 7 is a graphical representation for comparison of antibody responses against HBsAg and HBV core antigen induced in Balb/c mice by HB-100 and HBV-110 DNA vaccines.
FIG. 8 is a graphical representation for comparison of cell-mediated immune responses specific to HBsAg, core antigen, PreS1/S antigen, and polymerase antigen induced in Balb/c mice by HB-100 and HB-110 DNA vaccines.
FIG. 9 is a graphical representation for comparison of proliferation efficacy of IL-12m on antibody responses and cell-mediated immune responses in Balb/c mice groups with and without IL-12m of HB-110.
FIG. 10 is a graphical representation for comparison of HBsAg-specific antibody responses induced in HBV transgenic mice by HB-100 and HB-110 DNA vaccines.
FIG. 11 is a graphical representation for comparison of cell-mediated immune responses specific to HBsAg and PreS1/S2 antigen induced by HB-100 and HB-110 DNA vaccines in HBV transgenic mice.
FIG. 12 is a graphical representation of cell-mediated immune responses specific to multiple antigens induced in HBV transgenic mice by injecting HB-110 once, twice and three times, respectively.
FIG. 13 is a graphical representation of the therapeutic effect of IL-12m as determined by a decrease in the serum HBsAg level when adding IL-12 variant to HBV transgenic mice.

### [Best Mode]

In order to achieve the objects, in one aspect of the present invention, there is provided a DNA vaccine comprising antigenic genes encoding hepatitis B surface antigen (HBsAg), L protein, a core, and a polymerase, the antigenic genes codon-optimized to increase expression levels.

In other words, the present invention provides a DNA vaccine comprising multiple antigens, not a single antigen. In order to enhance the therapeutic effect of chronic HBV infection, the DNA vaccine of the present invention can induce an immune response specific to the multiple antigens. In particular, the DNA vaccine of the present invention uses a core, which is a structural gene of HBV, and a polymerase, which is a non-structural gene, in addition to the HBV surface gene, like in the conventional DNA vaccine.

In addition, the DNA vaccine of the present invention considerably increases expression levels of the multiple antigens through codon optimization. It is commonly known that a limited expression level of viral protein results from an insufficient amount of tRNA species of viral codons in a host, which is one of ways to avoid host defense mechanism (J Mol Biol. 1982 158:573). In order to increase expression levels of viral genes in human body, many attempts are being made to provide high expression levels of codons prevalent in human cells and high GC content. In particular, these attempts are primarily made with HIV, HPV or HCV models. It was confirmed that use of these model vaccines increased the expression levels, resulting in enhanced antibody and cell-mediated immune responses (J Virol. 2003 77:4928 J Virol. 2000 74:4839 Gene Ther. 2003 10:686). To date, no report has yet been proposed that expression levels of HBV models were increased through codon optimization. The present invention is directed to develop a DNA vaccine with an enhanced expression rate of antigen by codon-optimizing various antigenic genes of HBV.

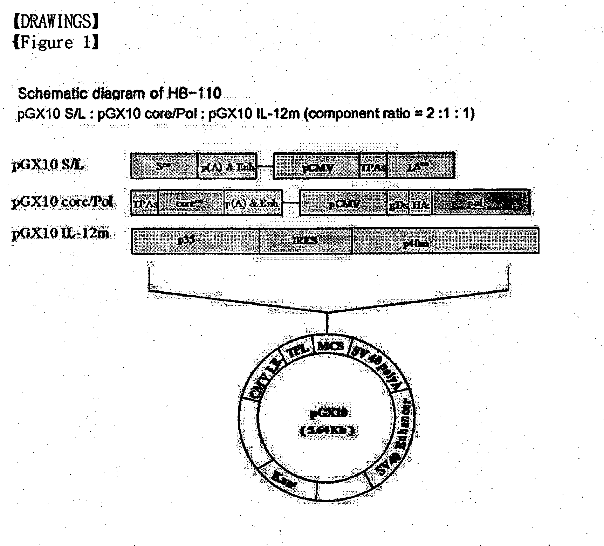

The DNA vaccine includes a plasmid (pGX10 S/L) encoding HBsAg antigen, which is a HBV surface gene, and pre-S1/S2/S (L protein) antigen, and a plasmid (pGX10 core/Pol) encoding a core antigen and a polymerase antigen (see FIG. 1).

The present invention is used as DNA vaccine for treatment of patients infected with chronic HBV, the inventors of the present invention developed two types of plasmids expressing HBV antigen of genotype C and named pGX10 S/L and pGX10 core/Pol, respectively.

The plasmid pGX10 S/L is an expression vector constructed such that the codon-optimized HBV S and L (large) glycoprotein (L protein) antigens are independently transcribed under the control of cytomegalovirus (CMV) promoters. The S antigen (M54923) was codon-optimized by GenScript Corp. (www.genescript.com) so as to be compatible to human. The L antigen (AF052576) was also codon-optimized by GenScript Corp. so as to be compatible to human. In addition, in order to facilitate extracellular secretion, signal sequence of human tissue-type plasminogen activator (tpa) was linked to the 5'-end of L antigen, and an amino acid at position No. 2, i.e., glycine, the secretion of which has been known to be suppressed by myristylation, was replaced by alanine. Then, the amino acids at position Nos. 91∼110, known to suppress the secretion of L antigen through allowing to retain L antigen in ER (Endoplasmic Reticulum), were eliminated. (Virology 1995 213: 57) (see FIG. 1).

The plasmid pGX10 core/Pol is an expression vector constructed such that genes of the core and polymerase (Pol) antigens are independently transcribed under the control of CMV promoters. The core antigen (M54923) was codon-optimized for gene synthesis by GenScript Corp. (www.genescript.com). In addition, similar to the case of the L antigen, in order to facilitate extracellular secretion, signal sequence of human tissue-type plasminogen activator (tpa) was linked to the 5'-end of the core antigen. The Pol antigen is an expression vector constructed in the same manner as pGX10 Pol (Gene Ther. 2006 13: 1110 - 1117), which is one of constituents of the therapeutic DNA vaccine HB-100 developed by the inventors of the present invention proposed in the previous research and synthesized by GenScript Corp., which will now be briefly described. In order to facilitate extracellular secretion, signal sequence (gDs) of glycoprotein gD of herpes simplex virus (HSV) was linked to the forefront 5'-end of the Pol antigen. In addition, in order to confirm expression, HA epitopes of influenza virus were inserted between gDs and Pol antigen (see FIG. 1).

The pGX10 vector used to develop the two plasmids is an expression vector having early promoter/enhancer sequences of cytomegalovirus (CMV) and adenovirus tripartite leader/intron, as indicated by TPL in FIG. 1, poly (A) sequence of SV40, as indicated by SVpA in FIG. 1, and has already been used as a DNA vaccine vector in small animal and clinical studies. More detailed explanation about the pGX10 vector is described in the previous application filed by the inventors of the present invention (Korean Patent Application No. 2003-0028080).

The present invention provides a DNA vaccine further including an immune adjuvant capable of enhancing cell-mediated immune responses, thereby facilitating the therapeutic effect of chronic HBV. Preferably, the present invention provides a DNA vaccine including a gene encoding interleukin 12 variant as the immune adjuvant.

The present invention provides a DNA vaccine including a plasmid (pGX10 IL-12m, see FIG. 1) expressing a gene encoding the interleukin 12 variant.

The interleukin-12 variant used as an immune adjuvant for the DNA vaccine was previously developed by the inventors of the present invention. Compared to a wild-type interleukin-12, the variant-type interleukin-12 induces long-lasting CD4 and CD8 T cell responses, which are specific to hepatitis C virus (HCV) antigen (Nat Biotechnol. 2002 20: 381, see Korean Patent No. 10-399728, etc.). More concretely, the interleukin-12 variant has an IL-12p40 subunit mutant gene which can secrete IL-12, but not p40 homodimer. Particularly, the expression vector including the IL-12p40 subunit mutant gene is constructed to inhibit the secretion of IL-12p40 but to normally secrete IL-12p70 that retains the immune activity of IL-12 by replacing the codon for Asn at the amino acid residue of 222 (human) or of 220 (mouse) of IL-12p40 with another codon. IL-12p40 is known to act as a competitive inhibitor of active form of IL-12, IL-12p70. In the present invention, the IL-12 variant having the above-described properties can be useful for HBV antigen specific immune responses and therapeutic effect.

Further, the present invention provides a method for preparing the DNA vaccine.

The multi-specific DNA vaccine having high expression rate of antigen and capable of effectively inducing cell-mediated immune responses is preferably prepared by a method comprising the steps of: (1) codon-optimizing antigenic genes encoding hepatitis B surface antigen (HBsAg), L protein, and a core; and (2) inserting the codon-optimized antigenic genes for the HBsAg, the L protein, and the core, and a gene encoding a polymerase, whether it has been either codon-optimized or not, to one or more expression vectors.

The genes encoding the codon-optimized HBsAg and L protein are inserted together to a single expression vector. Preferably, the two genes inserted together to the single expression vector are expressed by individual promoters, respectively. It is also preferable that the genes encoding the codon-optimized core and polymerase are inserted together to a single expression vector, and it is more preferable that these two genes inserted together to the single expression vector are expressed by individual promoters, respectively. Here, an appropriate expression vector known to those skilled in the art as along as it can be expressed in Eukaryotic cells. More preferably; pGX10 vector may be used as the expression vector, as disclosed in Korean Patent Application No. 2003-0028080 filed by the inventors of the present invention.

The method for preparing the DNA vaccine according to the present invention may further comprise the inclusion of an immune adjuvant. Any known immune adjuvant as long as it may induce cell-mediated immune responses and be safe is preferably used as the immune adjuvant of the present invention. Particularly, the immune adjuvant may be an expression vector comprising a gene encoding an IL-12 variant, which was previously developed by the inventors of the present invention. Here, the gene encoding an IL-12 variant is preferably mutated such that the codon for Asn, which is an amino acid residue 222 of human IL-12p40 represented by SEQ. ID. No 23, is substituted with another codon for a different amino acid, more preferably with the codon for Leu, Gln, or Ile. Alternatively, the gene encoding an IL-12 variant is preferably mutated such that the codon for Asn, which is an amino acid residue 220 of mouse IL-12p40, is substituted with another codon, more preferably with the codon for Leu or Ile. The pGX10 vector according to a preferred embodiment of the present invention may be used as the expression vector for expressing the IL-12 variant, but not limited thereto. The DNA vaccine according to the present invention can further facilitate induction of multi-specific T cell immune responses by further comprising the gene encoding IL-12 variant as an immune adjuvant.

Furthermore, the present invention provides a composition for use in curing chronic hepatitis B comprising the HBV vaccine according to the present invention as an active ingredient.

As described above, since the HBV vaccine according to the present invention induces multi-specific T cell immune responses, it can be effectively used as the vaccine for the treatment of the chronic HBV. Here, the vaccine of the invention may be used together with not only the gene encoding an IL-12 variant or other immune adjuvants but also a pharmaceutical carrier or excipient that may be widely used in the art.

The vaccine composition is formulated for humans or animals and can be administered through various routes including oral, intraperitoneal, intravenous, subcutaneous, intramuscular, intracutaneous, and intranasal administration. Preferably, the vaccine composition is formulated as an injection to be administered. The injection may be prepared using aqueous solvents such as saline solutions or Ringer solutions; or non-aqueous solvents such as vegetable oils, higher fatty acid esters (e.g., ethyl oleic acid, or the like), alcohols (e.g., ethanol, benzylalchol, propyleneglycol, glycerin, or the like). The injection may include pharmaceutical carriers, including a stabilizer for preventing degeneration, such as ascorbic acid, sodium bisulfite, sodium pyrophosphate, butylated hydroxyanisole (BHA), tocopherol, ethylenediamine tetraacetic acid (EDTA), or the like, an emulsifier, a buffering agent for adjusting a pH level, a preservative for prohibiting growth of microorganisms, such as phenylmercuric nitrate, thimerosal, benzalkonium chloride, phenol, cresol, benzyl alcohol, or the like.

The composition according to the present invention is administered in a pharmaceutically effective amount. The term "pharmaceutically effective amount" is a sufficient dose enough to demonstrate a vaccination effect or a small dose enough to prevent the occurrence of side effects or an excessive immune response. The pharmaceutically effective dose depends on the type of antigen administered, and other factors which those skilled in the medical arts will recognize, for example, patient' s age, weight, health condition, sex, drug sensitivity, administration route, or administration method. The composition according to the present invention may be administered at once, or may be divided into a number of smaller doses to be administered at varying intervals of time.

As described above, the HBV DNA vaccine according to the present invention comprising genes encoding codon-optimized HBV multiple antigens exhibit excellent antibody response and cell-mediated immune responses, as observed from naive mice and HBV transgenic mice models, compared with HB-100 vaccine previously prepared by the inventors of the present invention (that is, a DNA vaccine comprising genes encoding HBV core, S protein, polymerase, and PreS1/PreS2 which are not codon-optimized, and a gene encoding IL-12 variant, as immune adjuvants) (Gene Therapy, 2006 13: 1110 - 1117). Particularly, the HBV vaccine according to the present invention effectively induces HBV antigen specific immune responses and therapeutic effect. Particularly, it could be confirmed that the DNA vaccine according to the present invention could further facilitate induction of therapeutic responses by further comprising the gene encoding IL-12 variant as the immune adjuvant.

Therefore, the HBV DNA vaccine according to the present invention can be more effectively-used for the treatment of chronic hepatitis B than the conventional HBV DNA vaccine.

The invention will be described in greater detail by way of specific examples. The following examples are offered for illustrative purposes, and are not intended to limit the invention in any manner.

### [Mode for Invention]

### <EXAMPLE 1> Preparation of Plasmid constructing DNA Vaccine for Treatment of HBV

### EXAMPLE 1-1: Preparation of plasmid pGX10 S/L

First, in order to achieve codon optimization of HBsAg, a gene sequence with 5'-end EcoR I and 3'-end Not I was determined, gene of HBsAg (SEQ. ID. No 1) was synthesized by Genscript Corp., yielding a plasmid pUC57-S^{CO}. The plasmid pUC57-S^{CO} was digested with EcoR I and Not I, and pGX10) was also digested with EcoR I and Not I. These plasmids are linked together to finally construct pGX10-S^{co}. In constructing pGX10 S as a control, a gene without codon modification was synthesized by Genscript Corp., yieldinga plasmid pUC57-S in substantially the same manner as described above. In amino acid sequences of PreS1/S2, codon-optimized genes (SEQ. ID No 2), in which glycine was replaced by alanine and amino acids 91-110 were removed, were first synthesized by Genescript Corp. and plasmids pUC57-PreS1/S2^{co} were then obtained. TPAs (tissue plasminogen activator signal sequence) was formed such that two oligonucleotides TPA-1 represented by SEQ. ID No 3 and TPA-2 1 represented by SEQ. ID No 4 have EcoR I and Not I based on nucleotides, as published in PROTEIN EXPRES PURIF, 1998 14: 8∼12, so as to be complementarily combined with each other, thereby preparing double-stranded DNA. Thereafter, the prepared double-stranded DNA was inserted into pSK(+) vector (manufactured by Stratagene). Then, pSK(+)-TPAs was digested with EcoR I and Not I, pUC57-PreS1/S2^{CO} was digested with EcoR I and Not I, and the DNA fragments were then linked together, thereby obtaining pSK(+)-TPAs-PreS1/S2^{CO}.

In order to prepare L protein by combining TPAs-PreS1/S2^{CO} gene derived from pSK(+)-TPAs-PreS1/S2^{CO} with HBsAg antigenic gene, a primer Asp718TPAs (SEQ. ID No 5) including Asp 718 and Nco I restriction enzyme recognition sites and a Pst I S^{co} primer (SEQ. ID No 6) including Pst I restriction enzyme recognition site and 5'-end portion sequences of S^{CO}, were synthesized. PCR was performed using pSK(+)-TPAs-PreS1/S2^{CO} as a template, and the PCR product was digested with Asp718 and Pst I. The pGX10-S^{CO} vector was partially digested with Pst I, that is, only a Pst I site located within the S^{co} gene was digested while a Pst I site located at the pGX10 vector side is not digested. Then, the partially digested vector was digested with Asp 718, only a 5'-end portion of the S^{CO}-gene was removed, followed by linking together using only the vector portion, thereby preparing pGX10-TPAs-L^{CO}. Next, in order to amplify an overall area of a CMV promoter of pGX10, inclusive of even an SV40 enhancer, Sal I CMVp (SEQ. ID No 7) and Sa1 I SVpA/Enh primer (SEQ. ID No 8) were synthesized. PCR was performed using pGX10 as a template, the PCR product was immediately linked with pGEM-T-Easy vector (manufactured by Promega), thereby constructing pGEM-CMV-TPL-MCS-SVpA/Enh. Then, pGEM-CMV-TPL-MCS-SVpA/Enh was digested with Asp 718 and Xho I, and the resultant fragments were linked with pGX10-TPAs-L^{CO} to construct pGEM-CMV-TPL-TPAs-L^{co}-SVpA/Enh. Finally, the vector was digested with Sal I and a CMV-TPL-TPAs-L^{co}-SVpA/EnhS^{CO} fragment was inserted into the restriction site of the vector, produced by digesting pGX10 S^{co} with Sal I, thereby completing the target plasmid pGX10 S/L simultaneously expressing S and L antigens using dual promoters within a single vector, in such a manner that genes are expressed using CMV promoters positioned at 5'-end portions of the respective genes.

### EXAMPLE 1-2: Preparation of plasmid pGX10 core/Pol

Based on TPAs sequence and amino acid sequence of core protein, which has been described in EXAMPLE 1-1, a gene in the form of fusion protein was synthesized by Genscript Corp., yielding a plasmid pUC57-TPAs-core^{CO} (SEQ ID. No 9) with 5'-end EcoR I and 3'-end Not I and Xho I restriction enzyme recognition sites. The plasmid pUC57-TPAs-core^{CO} was digested with EcoR I and Xho I, and pGX10 was also digested with EcoR I and Xho I. These plasmids are linked together to finally construct pGX10-TPAs-core^{CO} (SEQ ID. No 9). In constructing pGX10 core without codon optimization to be used as a control and pGX10 core with codon optimization and unfused TPAs, the respective genes were synthesized by Genscript Corp., yielding the target genes by cloning in substantially the same manner as described above.

In order to express Pol antigen, gene synthesis was designed such that glycoprotein signal sequence (gDs, AAN74642) and HA epitope (AAB01168) of herpes simplex virus (HSV) were linked to an N-terminal area of a Pol antigenic gene using two glycine residues as linkers, as described in Gene Ther. 2006 13: 1110 - 1117. Pst I was formed at 5'-end and Not I was formed at 3'-end, and gene synthesis was made by Genscript Corp., thereby acquiring pUC57-gDs-HA-Pol with gDs-HA-Pol(SEQ. ID No 10) inserted into the pUC57 vector. The acquired pUC57-gDs-HA-Pol was digested with Pst I and Not I, pGX10 was digested with Pst I and Not I, and the resultant fragments were linked together, thereby constructing pGX10-gDs-HA-pol.

To construct pGX10 core/Pol, pGEM-CMV-TPL-MCS-SVpA/Enh was digested with Pst I and Xba I, pGX10-gDs-HA-Pol was digested with Pst I and Xba I, and the resultant fragments were linked together, thereby constructing a vector pGEM-CMV-TPL-gDs-HA-Pol-SvpA/Enh. Finally, the constructed vector pGEM-CMV-TPL-gDs-HA-Pol-SvpA/Enh was digested with Sal I and a CMV-TPL-gDs-HA-Pol-SvpA/Enh portion was inserted into a portion obtained by digesting pGX10-TPAs-core^{CO} with Sal I, thereby completing the plasmid pGX10 core/Pol simultaneously expressing core and Pol antigens using dual promoters within a single vector.

### EXAMPLE 1-3 : Preparation of plasmid pGX10 IL-12m

Using RT-PCR (PCR System 2400, Perkin Elmer) with complementary primers from NC37 cell, human B cell activated with PMA (phorbol myristic acetate), cDNAs (AF180563) encoding human p35 subunit of 820 bp and human p40 subunit of 1,050 bp were obtained. hp35 (S) (SEQ. ID No 11) and hp35 (AS) (SEQ. ID No 12) were used in amplifying the human p35 subunit, and hp40 (S) (SEQ. ID No 13) and hp 40(AS) (SEQ. ID No 14) were used in amplifying the human p40 subunit. Each amplified cDNA was subcloned into vector pBluescriptSK+ (Stratagene). Each of genes encoding P35 and p40 subunits was inserted into SmaI site of the vector pBluescriptSK+, thereby preparing pSK-hp35 (3.8kb) and pSK-hp40 (4.1kb).

In order to construct a bicistronic vector co-expressing the genes encoding p35 and p40 subunits, the IRES gene of EMCV was produced by RT-PCR and digested with restriction enzyme EcoR V. The resulting DNA fragment was inserted into EcoR V site of vector pSK to form a vector pSK- IRES of 3. 6kb. IRES (S) (SEQ. ID No 15) and IRES (AS) (SEQ. ID No 16) were used for amplification of IRES. Plasmid pSK-hp35 was digested with restriction enzymes EcoR V and Not I and filled with T4 DNA polymerase to obtain hp35 fragment of 0.8kb. This DNA fragment was inserted into DNA fragment of 3.6kb formed by digesting vector pSK-IRES with restriction enzyme EcoR V to construct pSK-hp35/IRES of 4.4kb. Plasmid pSK-hp35/IRES was digested with restriction enzymes Sma I and Cla I and ligated with a ligase so that the portion of the restriction enzyme site upstream of hp35 was removed. The hp40 DNA fragment of 1.1kb obtained by digesting pSK-hp40 with restriction enzymes Nco I and Not I was inserted into pSK-hp35/IRES to construct a plasmid that co-expresses the genes encoding p35 and p40 subunits. The thus obtained plasmid is designated pSK-hp35/IRES/hp40.

Primary PCR was carried out using T7(S) (SEQ. ID No 17) primer and hp40-N222L antisense(AS) (SEQ. ID No 18) primer and pSK-hp40 as template to substitute amino acid 222, asparagine, of hp40 with leucine. Similarly, secondary PCR was conducted using T3(AS) (SEQ. ID No 19) primer and hp40-N222L sense(S) (SEQ. ID No 20) primer. As the result, two PCR fragments sharing common site including mutational point were formed. The third PCR was carried out using a mixture of those fragments as template and T7(S) primer and T3(AS) primer to produce fusion product thereof. The resulting fusion DNA fragment was digested with restriction enzymes NcoI and NotI and inserted into vector pBluescriptSK+ of 3. 0kb, which had been digested with restriction enzyme SmaI, to construct plasmid pSK-hp40-N222L of 4. 1kb. hp40-N222L fragment of plasmid pSK-hp40-N222L was obtained using the restriction enzymesNco I and Not I and substituted with hp40 fragment of plasmid pSK-hp35-IRES-hp40, thereby preparing plasmid pSK-hp35-IRES-hp40-N222L of 5.5 kb. Finally, in order to transfer hp35-IRES-hp40-N222L fragment to pGX10 vector, PCR amplification was carried out using hIL-12(S) (SEQ. ID No 21) and hIL-12((AS) (SEQ. ID No 22) as primers and vector pSK-hp35-IRES-hp40-N222L as template, thereby obtaining fragment hp35-IRES-hp40-N222L. This fragment was digested with restriction enzyme XhoI and inserted into vector pGX10, which had been digested with restriction enzyme XhoI, to construct plasmid pGX10-hIL-12m (or pGX10-hIL-12N22L) of 6.1kb.

### <EXAMPLE 2> Confirmation of Expression Efficiency of HBsAg and Core Antigen by Codon Optimization

The inventors of the present invention attempted to confirm the expression efficiency of the antigens encoded by the plasmids prepared by using an in-vitro cell culture system, For this purpose, we used COS-7 cells (ATCC, CRL-1651), C2C12 cells (ATCC, CRL-1772), and HeLa cells (ATCC, CCL-2).

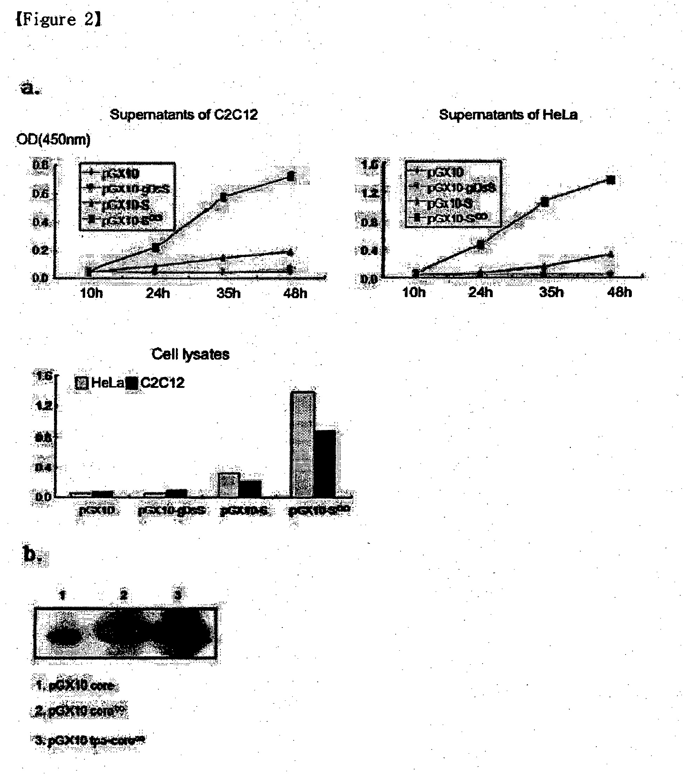

To confirm the expression efficiency of HBsAg, pGX10 gDsS, pGX10 S and pGX10 S^{co} were transfected with pGX10 and pGL2 luc (Promega) as control vectors by a lipofection method (Invitrogen), thereby acquiring supernatant and cells, which will now be briefly described. One day before transfection, the cells were plated on each 60 mm-dish to confluency of about 80-90%. On the day of transfection, 0.05 µg of DNA and 20 µl of lipofectamine were added to 0.5 ml of serum free media (DMEM) (Biowhittacker, Cambrex) in two sets of eppendorf tubes, and then stirred gently, followed by allowing the mixed solution to stand undisturbed at room temperature. After 30 minutes, the resultant mixed solution was dispensed on the cells. 6 hours after transition, the resultant product was washed with serum free DMEM media twice, and the culture media were changed freshly, that is, from the serum free DMEM media into DMEM media containing 5% FBS (fetal bovine serum). Supernatants were taken 10, 24, 35 and 48 hours after transfection. The cells were harvested 48 hour later and placed in a 1 ml cell culture lysis buffer (Promega) to collect cell lysates. In order to normalize a variation of transfection efficiency, that is, in order to removea variable which may be caused by a difference in transfection efficacy between plasmids, 0.05 µg of luciferase gene was mixed with the prepared plasmids before the transfection, and cell lysate and supernatant having substantially the same luciferase activity level were taken for ELISA assay. Then, expression levels of HBsAg were measured using an HBsAg ELISA kit (Dong-A Pharm.). To be summarized, a required amount of strip was taken and fixed on a frame, and a concentrated conjugate solution and a diluted conjugatesolution were mixed in a ratio of 1:25. 100 µl of a sample solution was placed in the respective wells of the plate, and 25 µl of a preformed conjugate solution was placed to each well, which was then reacted at 37 for 90 minutes. Before completion of the reaction, a substrate solution was diluted by a substrate buffering solution (1:100), and washed with a washing solution 5 times. A surplus washing solution was completely removed using a hygroscopic sheet, and 100 µl of a preformed substrate solution was placed, followed by reacting at room temperature. As a result, as confirmed from FIG. 2A, in both the cell lysates and supernatants of HeLa cell and C2Cl2 cell, the expression level of pGX10 S^{co} having codon optimized HBsAg gene was much higher than that of pGX10 S having a wild-type HBsAg gene. In addition, the expression level of pGX10 S^{co} was higher than that of pGX10 gDsS of HB-100 vaccine, which was previously developed by the inventors of the present invention, as described in EXAMPLE 3.

To confirm the expression of core antigen, the procedure was carried out in substantially the same manner as the above-described transfection. That is to say, pGX100-core, pGX10-core^{co}, and pGX10 tpa-core^{co} were transacted into HeLa cells by a lipofection method (Invitrogen). To perform RIP (radioimmunoprecipitation), used media were all removed, and the cells were allowed to stand in culture media DMEM lacking fetal bovine serum -(FBS), methionine (Met) and cysteine (Cys) for 30 minutes. After removing the DMEM media, incubation was carried out in media supplemented with 100 mCi [³⁵S]Met and 1% dialysed FBS for 4 hours. After washing with ice-cold PBS twice, the cells were treated with 0.5 ml of cell lysis buffer and placed on ice for 20 minutes. After collecting cells, the cells were sheared using a 1 cc syringe several times, subjected to centrifugation, thereby acquiring supernatants (cell lysates). Then, a volume of NET gel buffer equivalent to the quantity of the sample was added to each sample of the acquired supernatants (cell lysates), and 3 µl of HBV patient antisera was added thereto, followed by incubating at 4 for 6 hours. After addition of 30 µl of protein A-G agarose gel, incubation was further performed at 4 for 2 hours. After centrifugation, the supernatant was removed and 1 ml of RIPA buffer was added for vortexing, thereby resuspending the resultant solution. After washing with washing buffer (10 mM Tris-HCl (pH7.5), 0.1% NP-40) 4 times, 30 µl of 1X SDS gel loading buffer was added to the resultant solution and then boiled for 5 minutes. After centrifugation, the supernatant was loaded for SDS-PAGE(sodium dodecyl sulphate-polyacrylamide gel electrophoresis). After running the electrophoresis sufficiently, the gel was dipped in salicylic acid for 30 minutes and dried to be exposed to a film. As a result, as confirmed from FIG. 2B, pGX10 core and pGX10 tpa-core^{co} each having codon optimized HBsAg gene had increased expression levels compared to pGX10 core.

### EXAMPLE 3> Confirmation of Expression of Antigen by HB-110 therapeutic vaccine

The inventors of the present invention attempted to confirm expression efficiency of each antigen in HB-110 containing pGX10 S/L, pGX10 core/Pol, and pGX10 IL-12m.

To confirm the expression efficiency, HB-110 and HB-100 as a comparison group were transfected into C2C12 cell, HeLa cell or COS-7 cell. HB-110 was prepared by mixing 50% of pGX10 S/L, 25% of pGX10 core/Pol, and 25% of pGX10 IL-12m and a total amount of HB-110 used was 2 µg. HB-100 is a DNA vaccine previously prepared by the inventors of the present invention, as disclosed in Gene Therapy, 2006 13: 1110 - 1117, that is, a combination of 25% of pGX10 gDsS, 18.75% of pGX10 S1/S2/X, 18.75% of pGX10 core, 12.5% of pGX10 Pol, and 25% of pGX10 IL-12m and a total amount of HB-100 used was 2 µg. In pGX10 S1/S2/X plasmid, X denotes an X gene of HBV, i.e., HBx.

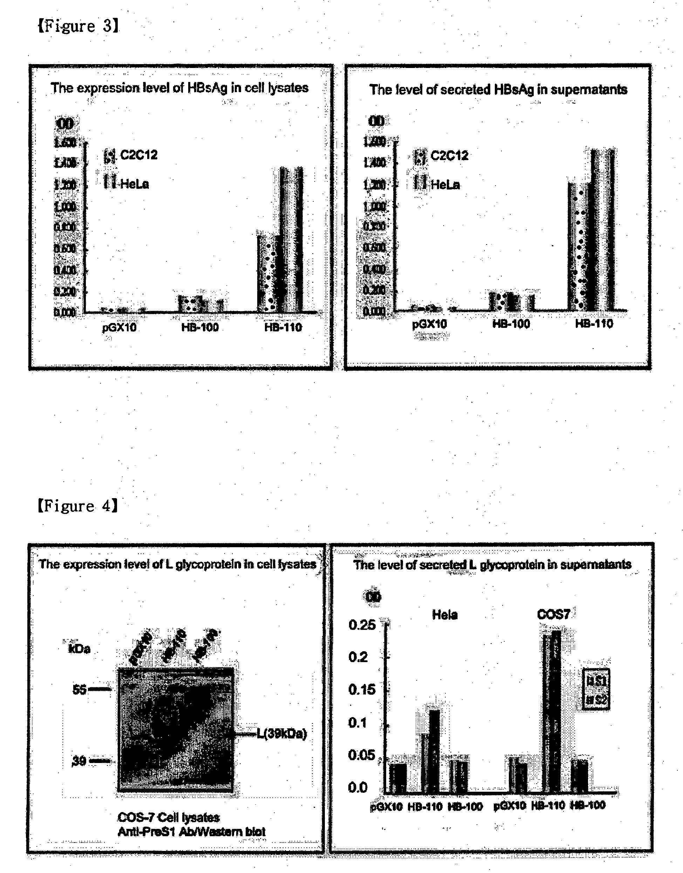

To confirm the expression of HBsAg from HB-110 and HB-100, C2C12 cell and HeLa cell were used. As described above, the C2C12 cell and the HeLa cell were cultured in DMEM containing 10% FBS (Gibco-BRL, USA). 5 X 10⁵ cells were distributed in a 60 mm plate, cells were grown for 24 hours, and then transfected into 2 µg of each of HB-110 and HB-100 using lipofectamine (Invitrogen). 48 hours after lipofection, the cells were harvested for ELISA assay. Normalization of the variation of transfection efficiency is performed in the above-described manner. That is to say, luciferase gene was mixed together before the transfection, and cell lysate and supernatant having substantially the same luciferase activity level were taken for ELISA assay. As confirmed from FIG. 3, in both cell lysate and supernatant, the HBsAg expression efficiency of HB-110 was higher than that of HB-100, which was developed by previously prepared by the inventors of the present invention.

To confirm the expression efficiency of L glycoprotein, 2 µg of each of HB-110 and HB-100 were transfected to COS-7 cell and HeLa cell using lipofectamine (Invitrogen). 48 hours after lipofection, the cells were harvested. Cell lysate having substantially the same luciferase activity level was taken for electrophoresis. After performing electrophoresis in 10% SDS-polyacrylamide gel, the resultant product was subjected to immunoblotting. An anti-PreS1 monoclonal antibody (Aprogen Co.) was used to detect a pre-S1/S2 protein. As shown in the left view of FIG. 4, HB-110 exhibited enhanced expression efficiency. The full-form of L protein encoded by HB-110 was detected by ELISA assay. In order to detect L protein, anti-PreS1 or anti-PreS2 monoclonal antibody (Aprogen Co.) as a capture antibody (capture Ab) was diluted in 1000 times, and an HRP-eonjugated ant-HBsAg polyclonal antibody contained in an HBsAg ELISA kit (Green-Cross) was used. As shown in the right view of FIG. 4, HB-110 and HB-100 exhibited the expected results, that is, HB-110 confirmed the expression of L protein in both HeLa and COS-7 cells while HB-100 as well as pGX10-transfected sample hardly expressed L-protein.

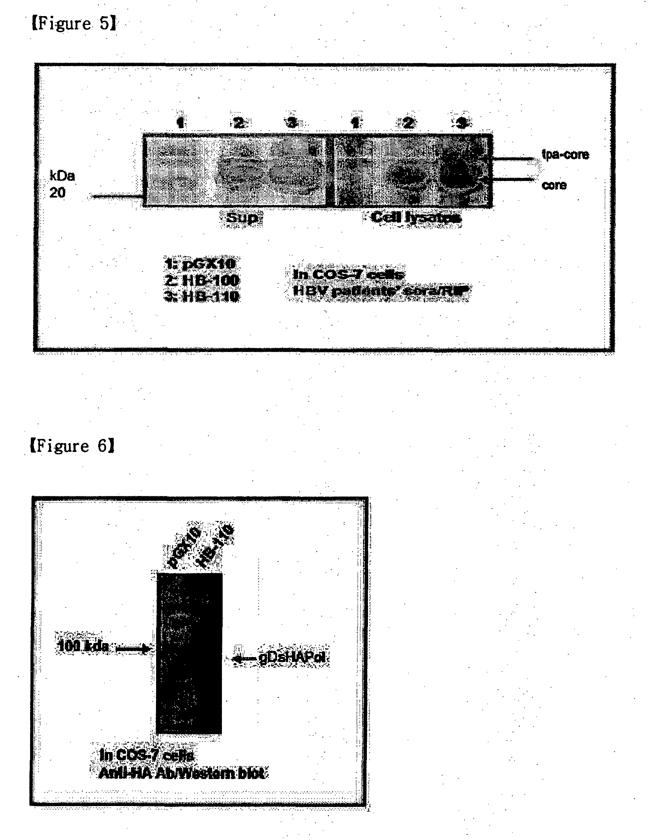

To confirm the expression of core proteins in HB-110 and HB-100, COS-7 cells were used. The COS-7 cells were transfected in the above-described manner, 48 hours after transfection, the cells were harvested. Cell lysates having substantially the same luciferase activity level were taken for electrophoresis, and RIP (radioimmunoprecipitation) was then performed. To avoid non-specific coupling,pre-clearing was performed using healthy human serum. To detect a core protein, RIP was performed with chronic hepatitis patient 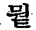 serum. As a result, as shown in FIG. 5, core protein generate by HB-110 was detectedin both cell lysate and supernatant and the detected level of HB-110 was slightly higher than that of HB-100.

To confirm the expression of a Pol antigen in HB-110, COS-7 cells were transfected in the above-described manner, 48 hours after transfection, the cells were harvested and cell lysates were taken for electrophoresis, followed by immunoblotting. Since an antibody capable of directly detecting a polymerase protein is not available, an HA epitope was linked to the 5'-end of a polymerase, and the expression of the polymerase protein was detected using an anti-HA monoclinic antibody, as confirmed from FIG. 6. The expression levels of Pol antigen in HB-110 and HB-100 were substantially the same with each other(data not shown). This is thought to be due to the Pol antigen in the form of gDs-HA-Pol included in the expression vector pGX10.

### <EXAMPLE 4> Evaluation of Immunogenicity of HB-110 in Mice

The inventors of the present invention carried out experiments for immunization of small animals. To this end, HB-100 and HB-110 were dissolved in 200µl of PBS, respectively, to be used as DNA vaccine. The first administration was performed by injecting 200 µl of a DNA vaccine into both legs (100 µl per each hind leg muscle) of 6 Balb/c mice per each group. The second and third intramuscular injections followed at 2 weeks interval. In case of HB-100, 25 µg of pGX10 gDsS, 18.75 µg of pGX10 S1/S2/X, 18.75µg of pGX10 core, 12.5 µg of pGX10 Pol, and 25 µg of pGX10 IL-12m were used. In case of HB-110, 50 µg of pGX10 S/L, 25 µg of pGX10 core/Pol, and 25 µg of pGX10 IL-12m were used. To investigate antibody responses induced after immunisation, the respective mice were anesthetized at each administration time, and blood was collected from the infraorbital vein of each mouse using a heparinized capillary tube. To investigate cell-mediated immune responses, 2 weeks after the last administration, spleen cells were collected to be used for experiments.

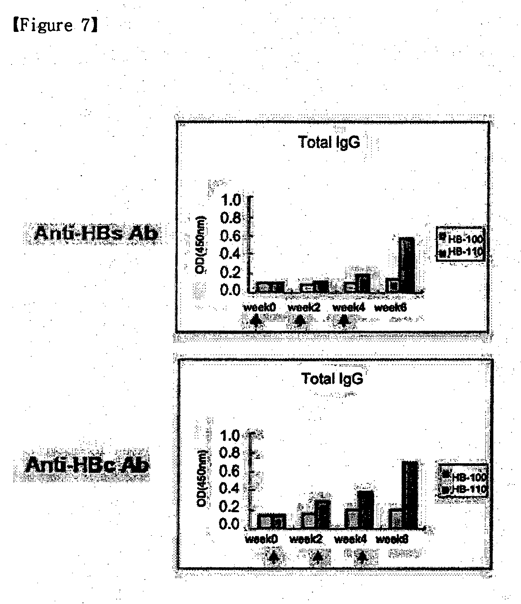

To investigate antibody responses, blood collected from each mouse was let to stand at room temperature for one hour. Then, after 5 minute centrifuging at 10,000 rpm, only the supernatant was taken to acquire serum. First, HBsAg (Fizgerald Ab&Ag, USA) was diluted in a concentration of 0.1 µ g/ml, HBcAg (Koma biotech. USA) was diluted in a concentration of 3 µg/ml, and these plasmids were distributed to different 96-well plates (Nunc, F96 immunoplate) by 50 µl each, to then be left at 37 for 2 hours. The contents in each 96-well plate were poured out, and washed 3 times with a PBS washing solution (0.05% PBS-T manufactured by GibcoBRL) containing 0.05% tween 20 (Sigma). The mouse serum was diluted with by a buffer solution(1:50), prepared by adding 5% skim milk to the washing solution, and added to each well by 50 µl, which was then reacted at 37 for 1.5 hour. On completion of the reaction, followed by washing with a washing solution (PBS-T, 0.05%) 5 times, HRP-conjugated anti-mouse immunoglobulin (anti-total IgG) antibodies were diluted with a buffer solution in 3000 times and distribute to each well by 50 µl, followed by reacting at room temperature for one hour. Then, after washing with testing buffer solution 5 times, each 50 µl of a 1:1 mixed solution of tetramethylbenzidine (TMB) substrate (manufactured by KPL) and peroxidase substrate solution B (manufactured by KPL) was added to each well and allowed to stand undisturbed at room temperature. When the color of each well turns to blue, each 50 µl of 2N H₂SO₄ was distributed to each well to stop the color reaction. The absorbance of each well was measured at 450 nm using a microplate reader. As shown in FIG. 7, when HB-110 was injected, the anti-HBs antibody response and anti-HBc antibody response were induced efficiently in proportion to the number of injection times, while the antibody response was very weak in mice administered with HB-100.

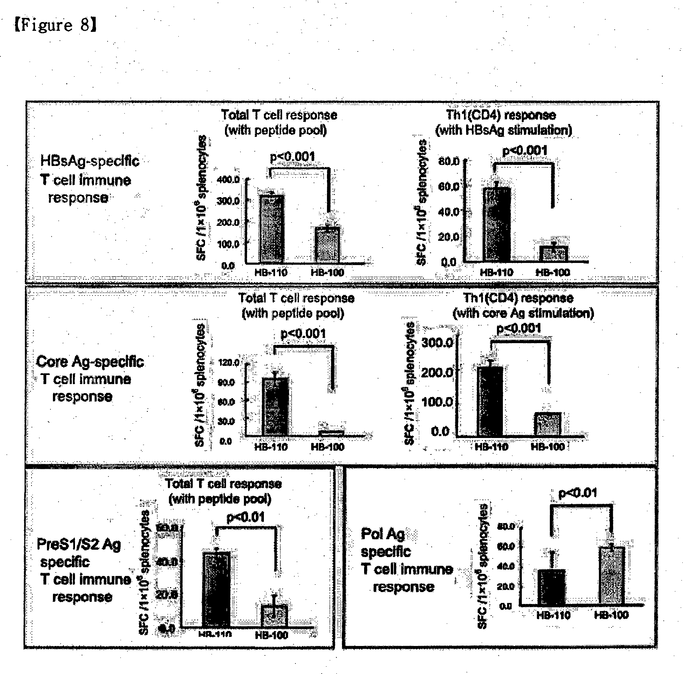

IFN-g ELISPOT analysis was repeatedly performed twice after pooling spleen cells taken from 3 immunized mice. Particularly, the coating antibody (BD Pharmingen) for IFN-g was diluted with PBS in a concentration of 5 µ g/ml, and 50 µl of the diluted antibody solution was distributed into a 96-well plate -(Millipore, 0.45m, Bedford, MA), which was then left at room temperature for over 12 hours. The remaining antibody solution was removed by suction. The plate was washed with PBS twice, and 200 µl of a medium for animal cell culture (RPMI-1640 containing 50 units/ml penicillin, 50 µg/ml streptomycin, 50 µM-mercaptoethanol, 2 mM L-glutamine, 1 mM sodium pyruvate, 20 units/ml recombinant murine IL-2 and 10% FBS (standard fetal bovine serum, HyClone) was added into each well. After allowing the plate to stand at 37 for over 2 hours, the medium was removed by suction again. The isolated spleen cells were added into each well by 1x10⁶, 3.3x10⁵ and 1.1x10⁵ cells/well, respectively. In order to investigate the HBV antigen specific immune response, 20-mer peptides (Peptron, Korea) were synthesized with 10-amino-acid overlaps based on amino acid sequences of antigens of HB-110. The synthesized peptides were mixed by antigen and added to each to finally reach 1 µg/well. Then, the 96-well plate was cultured at 37, 5% CO₂ incubator without agitation for 20 hours. After incubation, contents in the 96-well plate were poured out, and washed with a PBS washing solution containing 0.05% tween 20 (Sigma)(PBS-T, 0.05%) three times. Biotin-conjugated anti-IFN-g mAb (BD Pharmingen) was diluted with blocking buffer, prepared by adding 1% BSA to the washing solution, to adjust the concentration to 2 µg/ml, which was then added into each well by 50 µl, followed by reacting for 2 hours. After washing with the washing solution (PBS-T, 0.05%) 5 times, the plate was filled with streptavidin-AKP solution diluted again by the blocking buffer (1:2000), which was then reacted for 1 hour. Upon completion of the reaction, washing was performed with washing solution (PBS-T, 0.05%) 7 times. A mixture of 66 µl of NBT (Promega) and 33 µl of BCIP (Promega) was added to 10 ml of an alkaline phosphate buffer, and 50 µl of the resultant solution was added to each well to induce color development. The color reaction was stopped using tap water when a required size spot was observed (10-30 minutes). The 96-well plate was dried at room temperature, and then the number of cells secreting IFN-g was measured by using a microscope. As shown in FIG. 8 showing the cell-mediated immune responses, when a group administered with HB-110 showed statistically significant enhancement in the spot forming cell (SFC) count which is specific to HBsAg, core, and PreS1/S2 antigen, compared to a group administered with HB-100. In addition, not only when a peptide pool was used as a stimulant but also when a HBsAg protein or a core protein was used as a stimulant, HB-110 showed increased SFC counts, suggesting that CD4 T cell responses were induced more efficiently by HB-110 than by HB-100. In case of cell-mediated immune responses specific to Pol antigen, both HB-110 and HBV-100 induced immune responses in similar levels, which is thought to be due to the Pol antigen in the form of gDs-HA-Pol included in the expression vector pGX10.

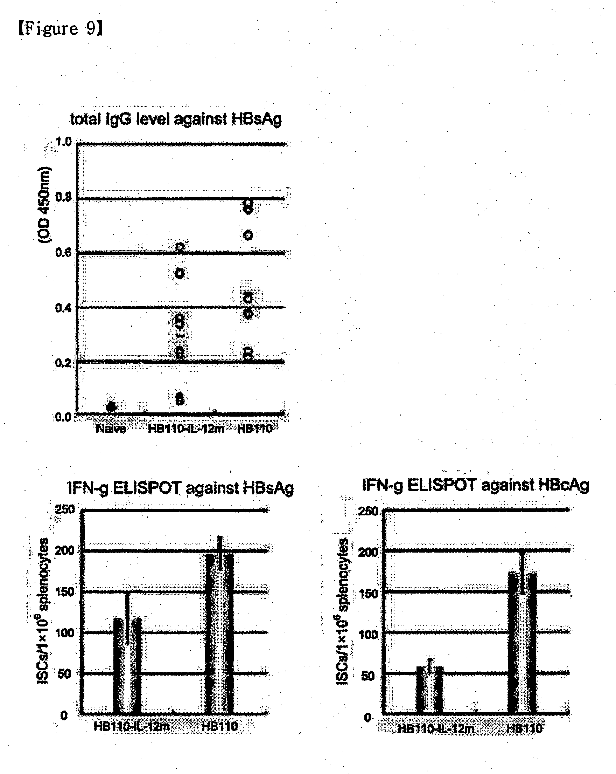

To confirm the effect of enhanced immunity of IL-12m among constituents of HB-110, 9 Balb/c mice per group were used in experiments. For experiment groups, administration was performed twice at a 2 week interval. That is to say, for one group, the first administration was performed by injecting 100 µl of HB-110 (50 µg of pGX10 S/L, 25 µg of pGX10 core/Pol, and 25 µg of pGX10 IL-12m). The second injection followed at a 2 week interval. For the other group, HB-110-IL-12m, which includes 25 µg of pGX10 vector, instead of 25 µg of IL-12m, was injected twice at a 2 week interval. On the second week after the second intramuscular injection, antibody and cell-mediated immune responses were induced in the above-described manner. As a result, as shown in FIG. 9, the group administered with HB-110 including IL-12m showed enhanced antibody and cell-mediated immune responses, compared to the HB-110-IL-12m without IL-12m, as observed by ELISA and IFN-g ELISPOT assays. More concretely, the HBsAg specific cell-mediated immune response was enhanced by about two times, and the cell-mediated immune response which is specific to HBcAg was enhanced by about three times. This suggests that IL-12m, which is one of constituents of HB-110 DNA vaccine, can effectively enhance antibody and cell-mediated immune responses when usedas an immune adjuvant.

### <EXAMPLE 5> Evaluation of Immunogenicity and Therapeutic effect of HB-110 in HBV transgenic mice

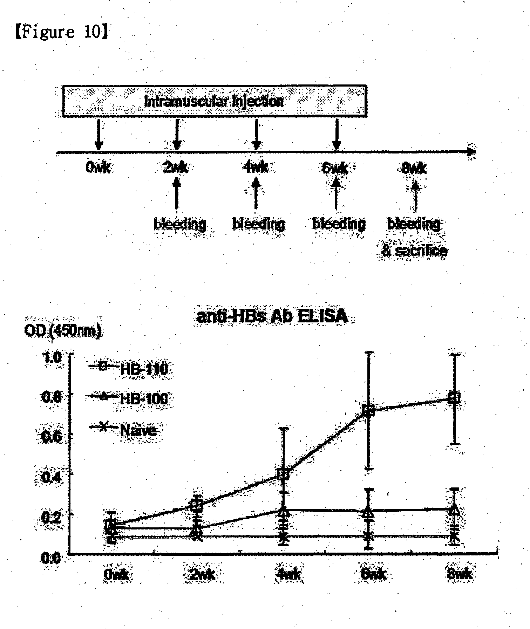

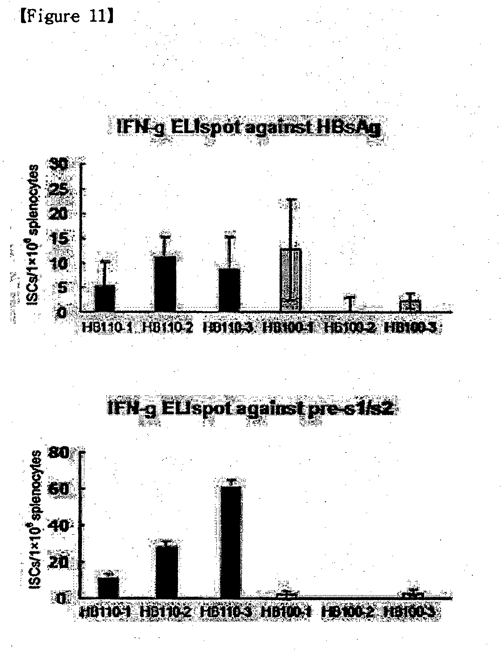

In order to evaluate the therapeutic effect of a DNA vaccine for HBV treatment, developed by the inventors of the present invention, HBV transgenic mice, in which replication of an HBV full genome is detected in liver cells, were used in evaluation (Proc. Natl. Acad. Sci. USA 1989, 86: 207). Like Balb/c mouse models, 100 µg of a DNA sample was dissolved in 200 ml of PBS, and the resultant solution was intramuscularly injected into the leg of each mouse. As shown in FIG. 10, 3 mice per group were used and the administration was made 4 times in total at 2-week intervals. Blood was collected from the infraorbital vein of each mouse at each administration time. 2 weeks after the last administration, spleen cells were collected to be used in measuring cell-mediated immune responses. The methods of determining the levels of the cell-mediated immune response and antibody response are the same as described in EXAMPLE 4. As shown in FIG. 10, similarly to the results observed from Babl/c mice, a relatively high level of anti-HBs antibody response was induced to HBV transgenic mice by HB-110, and the level of the anti-HBs antibody response induced was increased in proportion to the number of injection times. On the other hand, a very weak level of antibody response was induced to the HBV transgenic mice by HBV-100. As shown in FIG. 11, showing cell-mediated immune responses determined by IFN-g ELISPOT assay, the overall cell-mediated immune response, which is specific to HBsAg, was a weak level. In other words, 5∼12 ISCs (interferon gamma secreting cells) per 10⁶ splenocytes were detected from three mice administered with HB-110. Not greater than 5 ISCs per 10⁶ splenocytes were detected from two of three mice in a group administered with HB-100. Meanwhile, the cell-mediated immune response to PreS1/S2 significantly increased, unlike the cell-mediated immune response to HBsAg. In particular, the cell-mediated immune response, which is specific to preS1/S2, was efficiently induced to all three mice administered with HB-110. By contrast, three mice administered with HB-100 showed very weak immune responses, that is, not greater than 5 ISCs per 10⁶ splenocytes.

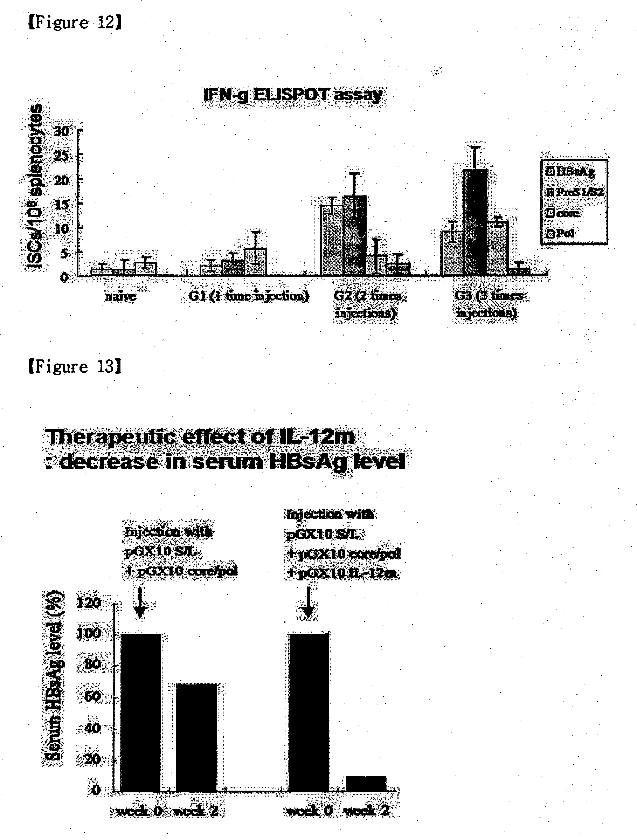

The HB-110 DNA vaccine includes multiple antigens. In this regard, the present invention aimed at investigating the capability of inducing each antigen specific cell-mediated immune response in HBV transgenic mice and the required number of injection times to induce significant immune responses. HB-110 DNA vaccine was injected to 3 mice per group once, twice and three times, respectively, and 2 weeks after HB-110 injection, spleen cells were collected to investigate cell-mediated immune responses. As a result, as shown in FIG. 12, as the number of injection times was increased, the overall cell-mediated immune response increased and a detectable level of immune response was induced by antigen. In case of a cell-mediated immune response, which is specific to HBsAg and PreS1/S2, the immune response induced was weak when HB-110 was injected once. By contrast, the immune response was better induced when HB-110 was injected twice and 3 times. In case of a cell-mediated immune response, which is specific to a core antigen, the immune response induced was weak when HB-110 was injected once and twice. By contrast, the immune response was better induced when HB-110 was injected 3 times. In case of a cell-mediated immune response, which is specific to a Pol antigen, an undetectable level of immune response was induced when HB-110 was injected once. Meanwhile, a weak immune response was induced when NB-110 was injected twice and 3 times.

In the mice immunized with HB-110, antibody and cell-mediated immune responses were well induced. To confirm the therapeutic effect of HB-110, the effect of reducing serum HBsAg levels are measured. In particular, HB-110 comprises IL-12m as an immune adjuvant. The present invention aimed at investigating the effect of IL-12m exerted on the therapeutic effect of HB-110. In addition, to investigate the expression level of serum HBsAg, which is a transgenic gene, an HBsAg ELISA kit (Green-Cross) was used. The expression level was quantified based on HBsAg purchased from Fizgerald Ab&Ag, and mouse serum was diluted with PBS in 50 times to be used as a sample. As shown in FIG. 13, when only a plasmid expressing pGX10 S/L and pGX10 core/Pol antigens was administered to the mice without IL-12m, the mice showed a decrease of approximately 30% in the serum HBsAg level. On the other hand, when a plasmid expressing these antigens was administered to the mice with pGX10 IL-12m injected into the plasmid in the form of HB-110, the mice showed a decrease of approximately 90% in the serum HBsAg level. Accordingly, the present invention proposes that IL-12m plays an important role in the therapeutic effect as confirmed in HBV transgenic mice when used with HB-110 DNA vaccine.

### [Industrial Applicability]

As described above, the present invention provides a DNA vaccine comprising antigenic genes encoding a hepatitis B surface antigen (HBsAg), a core antigen, and a polymerase antigen, so that not only an antibody response but also a cell-mediated immune response can be effectively induced immune responses, which are specific to multiple antigens, thereby providing a desirable preventive and therapeutic DNA vaccine demonstrating both preventive and therapeutic effects against chronic Hepatitis B. The genes included in the DNA vaccine preferably encode the antigens which have been codon-optimized so as to optimize the expression of antigens in mammals. Accordingly, the expression level of the DNA vaccine of the invention can be enhanced in mammals, thereby effectively inducing immune responses.
## Claims

1. A Hepatitis B virus (HBV) DNA vaccine comprising two expression vectors, wherein one expression vector comprises polynucleotide sequences encoding hepatitis B surface antigen (HBsAg) and L protein, and the other expression vector comprises polynucleotide sequences encoding core protein and polymerase, wherein the polynucleotide sequence encoding HBsAg comprises the polynucleotide sequence of SEQ. ID. NO: 1, the polynucleotide sequence encoding L protein comprises the polynucleotide sequence of SEQ. ID. NO: 2, the polynucleotide sequence encoding core protein comprises the polynucleotide sequence of SEQ. ID. NO: 9, and the polynucleotide sequence encoding polymerase comprises the polynucleotide sequence of SEQ. ID. NO: 10.

2. The HBV DNA vaccine of claim 1, wherein the polynucleotide sequences of the HBsAg, L protein and core protein encode codon-optimized antigens.

3. The HBV DNA vaccine of claim 1, wherein two polynucleotide sequences inserted together into a single expression vector are expressed by individual promoters, respectively.

4. The HBV DNA vaccine of claim 1, wherein the expression vector comprising polynucleotide sequences encoding HBsAg and L protein is pGX10 S/L vector as shown in fig 1 and the expression vector comprising polynucleotide sequences encoding core protein and polymerase is pGX10 core/Pol vector as shown in Fig 1.

5. The HBV DNAvaccine of claim 1, further comprising an immune adjuvant.

6. The HBV DNA vaccine of claim 5, wherein the immune adjuvant is a gene encoding IL-12 variant.

7. The HBV DNA vaccine of claim 6, wherein the IL-12 variant is mutated such that Asn, which is an amino acid residue 222 of human IL-12p40 represented by SEQ. ID. No 23, is substituted with a different amino acid.

8. The HBV DNA vaccine of claim 6, wherein the IL-12 variant is mutated such that Asn, which is an amino acid residue 220 of mouse IL-12p40 represented by SEQ. ID. No 24, is substituted with a different amino acid.

9. The HBV DNA vaccine of claim 7, wherein the different amino acid is selected from the group consisting of Leu, Gln and Ile.

10. The HBV DNA vaccine of claim 8, wherein the different amino acid is selected from the group consisting of Leu and Ile.

11. The HBV DNA vaccine of anyone of claims 1 to 9, further comprising a pharmaceutically acceptable carrier or excipient.

12. A method for preparing the Hepatitis B virus (HBV) DNA vaccine according to claim 1, comprising:
(1) codon-optimizing polynucleotide sequence encoding hepatitis B surface antigen (HBsAg), L protein, and a core protein; and
(2) inserting the codon-optimized polynucleotide sequences encoding the HBsAg and L protein together into a single expression vector and inserting the codon-optimized polynucleotide sequence encoding the core protein and polynucleotide sequence encoding a polymerase of HBV together into another expression vector.

13. The method of claim 12, wherein the expression vector comprising polynucleotide sequences encoding HBsAg and L protein is pGX10 S/L vector as shown in fig 1, and the expression vector comprising polynucleotide sequences encoding core protein and polymerase is pGX10 core/Pol as shown in fig 1.

14. The method of claim 12, wherein the two polynucleotide sequences inserted together to the single expression vector are expressed by individual promoters, respectively.

15. The method of claim 12, further comprising the inclusion of an immune adjuvant.

16. The method of claim 12, wherein the immune adjuvant is a gene encoding 1L-12 variant.

17. The method of claim 16, wherein the gene encoding 1L-12 variant is mutated such that the codon for Asn, which is an amino acid residue 222 of human 1L-12p40 represented by SEQ. ID. No 23, is substituted with another codon for a different amino acid.

18. The method of claim 16, wherein the gene encoding 1L-12 variant is mutated such that the codon for Asn, which is an amino acid residue 220 of mouse 1L-12p40 represented by SEQ. ID. No 24, is substituted with another codon for a different amino acid.

19. The method of claim 17, wherein the different amino acid is selected from the group consisting of Leu, Gln and Ile.

20. The method of claim 18, wherein the different amino acid is selected from the group consisting of Leu and Ile.

21. The method of claim 16 or 17, wherein the inclusion of an immune adjuvant that is a gene encoding 1L-12 variant into an expression vector.

22. The HBV DNA vaccine of anyone of claims 1 to 11 for use in a method of treating chronic Hepatitis B.

## Patentansprüche

1. Hepatitis-B-Virus(HBV)-DNA-Impfstoff, umfassend zwei Expressionsvektoren, wobei ein Expressionsvektor Polynukleotidsequenzen umfasst, die Hepatitis-B-Oberflächenantigen (HBsAg) und L-Protein kodieren, und der andere Expressionsvektor Polynukleotidsequenzen umfasst, die Kernprotein und Polymerase kodieren, wobei die Polynukleotidsequenz, die HBsAg kodiert, die Polynukleotidsequenz mit SEQ. ID. NO: 1 umfasst, die Polynukleotidsequenz, die L-Protein kodiert, die Polynukleotidsequenz mit SEQ. ID. NO: 2 umfasst, die Polynukleotidsequenz, die Kernprotein kodiert, die Polynukleotidsequenz mit SEQ. ID. NO: 9 umfasst, und die Polynukleotidsequenz, die Polymerase kodiert, die Polynukleotidsequenz mit SEQ. ID. NO: 10 umfasst.

2. HBV-DNA-Impfstoff nach Anspruch 1, wobei die Polynukleotidsequenzen von HBsAg, L-Protein und Kernprotein Codon-optimierte Antigene kodieren.

3. HBV-DNA-Impfstoff nach Anspruch 1, wobei zwei Polynukleotidsequenzen, die zusammen in einen einzigen Expressionsvektor eingefügt sind, jeweils von individuellen Promotern exprimiert werden.

4. HBV-DNA-Impfstoff nach Anspruch 1, wobei der Expressionsvektor, umfassend Polynukleotidsequenzen, die HBsAg und L-Protein kodieren, der Vektor pGX10 S/L ist, wie in Fig. 1 gezeigt, und der Expressionsvektor, umfassend Polynukleotidsequenzen, die Kernprotein und Polymerase kodieren, der Vektor pGX10 core/Pol ist, wie in Fig. 1 gezeigt.

5. HBV-DNA-Impfstoff nach Anspruch 1, ferner umfassend ein Immunadjuvans.

6. HBV-DNA-Impfstoff nach Anspruch 5, wobei das Immunadjuvans ein Gen ist, das die IL-12-Variante kodiert.

7. HBV-DNA-Impfstoff nach Anspruch 6, wobei die IL-12-Variante derart mutiert ist, dass Asn, bei dem es sich um einen Aminosäurerest 222 von humanem IL-12p40 handelt, dargestellt durch SEQ. ID. No: 23, mit einer anderen Aminosäure substituiert ist.

8. HBV-DNA-Impfstoff nach Anspruch 6, wobei die IL-12-Variante derart mutiert ist, dass Asn, bei dem es sich um einen Aminosäurerest 220 von murinem IL-12p40 handelt, dargestellt durch SEQ. ID. No: 24, mit einer anderen Aminosäure substituiert ist.

9. HBV-DNA-Impfstoff nach Anspruch 7, wobei die andere Aminosäure ausgewählt ist aus der Gruppe bestehend aus Leu, Gln und Ile.

10. HBV-DNA-Impfstoff nach Anspruch 8, wobei die andere Aminosäure ausgewählt ist aus der Gruppe bestehend aus Leu und Ile.

11. HBV-DNA-Impfstoff nach einem der Ansprüche 1 bis 9, ferner umfassend einen pharmazeutisch verträglichen Träger oder Exzipienten.

12. Verfahren zur Herstellung eines Hepatitis-B-Virus(HBV)-DNA-Impfstoffs nach Anspruch 1, umfassend:
(1) Codon-Optimieren der Polynukleotidsequenz, die Hepatitis-B-Oberflächenantigen (HBsAg), L-Protein und ein Kernprotein kodiert; und
(2) Einfügen der Codon-optimierten Polynukleotidsequenzen, die das HBsAg und das L-Protein kodieren, zusammen in einen einzigen Expressionsvektor und Einfügen der Codon-optimierten Polynukleotidsequenz, die das Kernprotein kodiert, und der Polynukleotidsequenz, die die Polymerase von HBV kodiert, zusammen in einen anderen Expressionsvektor.

13. Das Verfahren nach Anspruch 12, wobei der Expressionsvektor, umfassend Polynukleotidsequenzen, die HBsAg und L-Protein kodieren, der Vektor pGX10 S/L ist, wie in Fig. 1 gezeigt, und der Expressionsvektor, umfassend Polynukleotidsequenzen, die Kernprotein und Polymerase kodieren, pGX10 core/Pol ist, wie in Fig. 1 gezeigt.

14. Verfahren nach Anspruch 12, wobei die beiden Polynukleotidsequenzen, die zusammen in einen einzigen Expressionsvektor eingefügt sind, jeweils von individuellen Promotern exprimiert werden.

15. Verfahren nach Anspruch 12, ferner umfassend das Einfügen eines Immunadjuvans.

16. Verfahren nach Anspruch 12, wobei das Immunadjuvans ein Gen ist, das die IL-12-Variante kodiert.

17. Verfahren nach Anspruch 16, wobei das Gen, das die IL-12-Variante kodiert, derart mutiert ist, dass der Codon für Asn, bei dem es sich um einen Aminosäurerest 222 von humanem IL-12p40 handelt, dargestellt durch SEQ. ID. No: 23, mit einem anderen Codon für eine andere Aminosäure substituiert ist.

18. Verfahren nach Anspruch 16, wobei das Gen, das die IL-12-Variante kodiert, derart mutiert ist, dass der Codon für Asn, bei dem es sich um einen Aminosäurerest 220 von murinem IL-12p40 handelt, dargestellt durch SEQ. ID. No: 24, mit einem anderen Codon für eine andere Aminosäure substituiert ist.

19. Verfahren nach Anspruch 17, wobei die andere Aminosäure ausgewählt ist aus der Gruppe bestehend aus Leu, Gln und Ile.

20. Verfahren nach Anspruch 18, wobei die andere Aminosäure ausgewählt ist aus der Gruppe bestehend aus Leu und Ile.

21. Verfahren nach Anspruch 16 oder 17, wobei ein Immunadjuvans, bei dem es sich um ein Gen handelt, das die IL-12-Variante kodiert, in einen Expressionsvektor eingeführt wird.

22. HBV-DNA-Impfstoff nach einem der Ansprüche 1 bis 11 zur Verwendung in einem Verfahren zur Behandlung chronischer Hepatitis B.

## Revendications

1. Vaccin à ADN du virus de l'hépatite B (VHB) comprenant deux vecteurs d'expression, dans lequel un vecteur d'expression comprend des séquences de polynucléotides codant pour l'antigène de surface d'hépatite B (AgHBs) et une protéine L, et l'autre vecteur d'expression comprend des séquences de polynucléotides codant pour une protéine capsidique et une polymérase, dans lequel la séquence de polynucléotides codant pour AgHBs comprend la séquence de polynucléotides de SEQ. ID No. : 1, la séquence de polynucléotides codant pour la protéine L comprend la séquence de polynucléotides de SEQ. ID No. : 2, la séquence de polynucléotides codant pour la protéine capsidique comprend la séquence de polynucléotides de SEQ. ID No. : 9, et la séquence de polynucléotides codant pour la polymérase comprend la séquence de polynucléotides de SEQ. ID No. : 10.

2. Vaccin à ADN de VHB selon la revendication 1, dans lequel les séquences de polynucléotides du AgHBs, de la protéine L et de la protéine capsidique codent pour des antigènes optimisés par codon.

3. Vaccin à ADN de VHB selon la revendication 1, dans lequel deux séquences de polynucléotides insérées conjointement dans un vecteur d'expression unique sont exprimées par des promoteurs individuels, respectivement.

4. Vaccin à ADN de VHB selon la revendication 1, dans lequel le vecteur d'expression comprenant des séquences de polynucléotides codant pour AgHBs et la protéine L est le vecteur pGXIO S/L tel qu'illustré sur la figure 1 et le vecteur d'expression comprenant les séquences de polynucléotides codant pour la protéine capsidique et la polymérase est le vecteur pGXIO core/Pol tel qu'illustré sur la figure 1.

5. Vaccin à ADN de VHB selon la revendication 1, comprenant en outre un adjuvant immunitaire.

6. Vaccin à ADN de VHB selon la revendication 5, dans lequel l'adjuvant immunitaire est un gène codant pour un variant d'IL-12.

7. Vaccin à ADN de VHB selon la revendication 6, dans lequel le variant d'IL-12 est muté de telle sorte qu'Asn, qui est un résidu d'acide aminé 222 d'IL-12p40 humain représenté par SEQ. ID No. : 23, est substitué par un acide aminé différent.

8. Vaccin à ADN de VHB selon la revendication 6, dans lequel le variant d'IL-12 est muté de telle sorte qu'Asn, qui est un résidu d'acide aminé 220 d'IL-12p40 de souris représenté par SEQ. ID No. : 24, est substitué par un acide aminé différent.

9. Vaccin à ADN de VHB selon la revendication 7, dans lequel l'acide aminé différent est choisi dans le groupe constitué de Leu, Gin et Ile.

10. Vaccin à ADN de VHB selon la revendication 8, dans lequel l'acide aminé différent est choisi dans le groupe constitué de Leu et Ile.

11. Vaccin à ADN de VHB selon l'une quelconque des revendications 1 à 9, comprenant en outre un véhicule ou excipient acceptable sur le plan pharmaceutique.

12. Procédé de préparation du vaccin à ADN du virus de l'hépatite B (VHB) selon la revendication 1, comprenant :
(1) l'optimisation par codon d'une séquence de polynucléotides codant pour l'antigène de surface d'hépatite B (AgHBs), une protéine L, et une protéine capsidique ; et
(2) l'insertion des séquences de polynucléotides optimisées par codon codant pour l'AgHBs et la protéine L conjointement dans un vecteur d'expression unique et l'insertion de la séquence de polynucléotides optimisée par codon codant pour la protéine capsidique et la séquence de polynucléotides codant pour une polymérase de VHB conjointement dans un autre vecteur d'expression.

13. Procédé selon la revendication 12, dans lequel le vecteur d'expression comprenant des séquences de polynucléotides codant pour AgHBs et la protéine L est le vecteur pGXIO S/L tel qu'illustré sur la figure 1 et le vecteur d'expression comprenant les séquences de polynucléotides codant pour la protéine capsidique et la polymérase est pGXIO core/Pol tel qu'illustré sur la figure 1.

14. Procédé selon la revendication 12, dans lequel les deux séquences de polynucléotides insérées conjointement dans le vecteur d'expression unique sont exprimées par des promoteurs individuels, respectivement.

15. Procédé selon la revendication 12, comprenant en outre l'inclusion d'un adjuvant immunitaire.

16. Procédé selon la revendication 12, dans lequel l'adjuvant immunitaire est un gène codant pour un variant d'IL-12.

17. Procédé selon la revendication 16, dans lequel le gène codant pour le variant d'IL-12 est muté de telle sorte que le codon pour Asn, qui est le résidu d'acide aminé 222 de l'IL-I2p40 humain représenté par SEQ. ID No. : 23, est substitué par un autre codon pour un acide aminé différent.

18. Procédé selon la revendication 16, dans lequel le gène codant pour le variant d'IL-12 est muté de telle sorte que le codon pour Asn, qui est le résidu d'acide aminé 220 de l'IL-I2p40 de souris représenté par SEQ. ID No. : 24, est substitué par un autre codon pour un acide aminé différent.

19. Procédé selon la revendication 17, dans lequel l'acide aminé différent est choisi dans le groupe constitué de Leu, Gin et Ile.

20. Procédé selon la revendication 18, dans lequel l'acide aminé différent est choisi dans le groupe constitué de Leu et Ile.

21. Procédé selon la revendication 16 ou 17, dans lequel on effectue l'inclusion d'un adjuvant immunitaire qui est un gène codant pour le variant d'IL-12 dans un vecteur d'expression.

22. Vaccin à ADN de VHB selon l'une quelconque des revendications 1 à 11 utilisable dans une méthode de traitement de l'hépatite B chronique.


<110>POSTECH FOUNDATION POSCO DONG-A PHARM.CO.,LTD Genexine Inc. DAEWOONG PHARMACEUTICAL CO.,LTD
<120> A DNA vaccine for curing chronic hepatitis B and a method of preparing same
<160> 24
<170> KopatentIn 1.71
<210> 1
   <211> 695
   <212> DNA
   <213> Artificial Sequence
<220>
   <223> HBsAg
<400> 1
   gaattcatgg agaacatcgc cagcggcctg ctgggccccc tgctggtgct gcaggccggc 60
   ttttttctgc tgaccaagat cctgaccatc ccccagagcc tggacagctg gtggaccagc 120
   ctgaactttc tgggcgggac gcctgtctgt ctggggcaaa acagccagag ccagatcagc 180
   agccacagcc ccacctgctg cccccccatc tgccccggct accgctggat gtgcctgcgc 240
   cgctttatca tctttctgtg catcctgctg ctgtgcctga tctttctgct ggtgctgctg 300
   gactaccagg gcatgctgcc cgtgtgcccc ctgatccccg gcagcagcac caccagcacc 360
   ggcccctgca agacctgcac cacccccgcc cagggcacca gcatgtttcc cagctgctgc 420
   tgcaccaagc ccaccgacgg caactgcacc tgcatcccca tccccagcag ctgggccttt 480
   gccaagtacc tgtgggagtg ggccagcgtg cgctttagct ggctgagcct gctggtgccc 540
   tttgtgcagt ggtttgtggg cctgagcccc accgtatggc tgagcgtgat ctggatgatg 600
   tggttttggg gccccagcct gtacaacatc ctgagcccct ttatgcccct gctgcccatc 660
   tttttttgcc tgtgggtgta catctaagcg gccgc 695
<210> 2
   <211> 563
   <212> DNA
   <213> Artificial Sequence
<220>
   <223> PreS1/S2, conflict(2, from 91 to 110)
<400> 2
   atggatgcta tgaaacgggg cctgtgctgc gtgctgctcc tgtgcggcgc tgtgtttgtg 60
   agccctagcg ctagcgaatt catggcaggg tggtcctcaa agccacgaca gggcatggga 120
   acaaatctgt cagtgcccaa tcctctcggc ttctttcccg accaccagct cgaccccgct 180
   ttcggggcca actctaacaa tcccgattgg gatttcaatc caaacaaaga ccattggccc 240
   gaggccaatc aggtgggcgt tggagccttc gggcccggct tcaccccccc tcatggcggg 300
   ctgcttgggt ggtctccaca ggcacaggga atcctgacta ccgtccctgc cggcttggaa 360
   tcgaattctc gcgattccca ccctcaggct atgcagtgga actctactac atttcaccag 420
   gccctgccag atccaagagt gcgggggctg tacttccccg caggcggaag cagttccggc 480
   accgtgaacc ccgtcccaac tacagcctct ccaattagct caattttttc acgcaccggc 540
   gaccccgccc ctaacgcggc cgc 563
<210> 3
   <211> 80
   <212> DNA
   <213> Artificial Sequence
<220>
   <223> TPA-1
<400> 3
   ggtaccatgg atgcaatgaa gagagggctc tgctgtgtgc tgctgctgtg tggagcagtc 60
   ttcgtttcgc ccagcgctag 80
<210> 4
   <211> 80
   <212> DNA
   <213> Artificial Sequence
<220>
   <223> TPA-2
<400> 4
   gaattcctag cgctgggcga aacgaagact gctccacaca gcagcagcac acagcagagc 60
   cctctcttca ttgcatccat 80
<210> 5
   <211> 26
   <212> DNA
   <213> Artificial Sequence
<220>
   <223> Asp 718 TPAs
<400> 5
   ggtaccatgg atgcaatgaa gagagg 26
<210> 6
   <211> 66
   <212> DNA
   <213> Artificial Sequence
<220>
   <223> primer
<400> 6
   ctgcagaaca agcagtggtc cgagcaggcc gctagcaata ttttccatgt taggggcggg 60
   gtcgcc 66
<210> 7
   <211> 23
   <212> DNA
   <213> Artificial Sequence
<220>
   <223> SalI CMVp
<400> 7
   ggtaccgtac cgatgtacgg gcc 23
<210> 8
   <211> 18
   <212> DNA
   <213> Artificial Sequence
<220>
   <223> SalI SV40 Enh
<400> 8
   gtcgacggat ccgctgtg
   18
<210> 9
   <211> 651
   <212> DNA
   <213> Artificial Sequence
<220>
   <223> tpa-coreCO
<400> 9
   gaattcgcca ccatggatgc tatgaaacgg ggcctgtgct gcgtgctgct cctgtgcggc
   60
   gctgtgtttg tgagccctag cgctatggac attgacccct acaaagaatt tggagcttct
   120
   gtggagctgc tctcttttct gccttctgac ttctttcctt ctattcggga tctgctcgat
   180
   acagcttctg ctctgtaccg ggaagctctg gaatctcctg aacactgcag ccctcaccac
   240
   acagcactcc ggcaggctat tctgtgctgg ggcgaactga tgaatctggc tacatgggtg
   300
   ggaagcaatc tggaagatcc agcatcccgg gaactggtgg tcagctacgt caatgtgaat
   360
   atgggcctga aaattcggca gctgctctgg tttcacattt cctgcctgac atttggacgg
   420
   gaaaccgtgc tggaatacct ggtgtctttt ggagtgtgga ttcggacacc tcctgcatac
   480
   cggccaccta atgctcctat cctgagcaca ctgcctgaaa caacagtggt ccggcggcgg
   540
   ggccggtccc ctcggcggcg gacacctagc cctcggcggc ggcggtctca gagccctggc
   600
   cggcggcggt ctcagtctcg ggaatctcag tgcgggcgcg cctagctcga g
   651
<210> 10
   <211> 2723
   <212> DNA
   <213> Artificial Sequence
<220>
   <223> gDs-HA-pol
<400> 10
<210> 11
   <211> 25
   <212> DNA
   <213> Artificial Sequence
<220>
   <223> hp35(S)
<400> 11 <210> 12
   <211> 17
   <212> DNA
   <213> Artificial Sequence
<220>
   <223> hp35(AS)
<400> 12
<210> 13
   <211> 27
   <212> DNA
   <213> Artificial Sequence
<220>
   <223> hp40 (S)
<400> 13
   agagcaccat gggtcaccag cagttgg
   27
<210> 14
   <211> 26
   <212> DNA
   <213> Artificial Sequence
<220>
   <223> hp40(AS)
<400> 14
   cgatgcggcc gcacctaact gcaggg
   26
<210> 15
   <211> 20
   <212> DNA
   <213> Artificial Sequence
<220>
   <223> IRES (S)
<400> 15
   aagatatcga attccccctc
   20
<210> 16
   <211> 21
   <212> DNA
   <213> Artificial Sequence
<220>
   <223> IRES(AS)
<400> 16
   ttgccatggc catatttatc a
   21
<210> 17
   <211> 22
   <212> DNA
   <213> Artificial Sequence
<220>
   <223> T7(S)
<400> 17
   gtaatacgac tcactatagg gc
   22
<210> 18
   <211> 21
   <212> DNA
   <213> Artificial Sequence
<220>
   <223> hp40-N222L(AS)
<400> 18
   tatgagctct acaccagcag c
   21
<210> 19
   <211> 20
   <212> DNA
   <213> Artificial Sequence
<220>
   <223> T3(AS)
<400> 19
   aattaaccct cactaaaggg
   20
<210> 20
   <211> 21
   <212> DNA
   <213> Artificial Sequence
<220>
   <223> hp40-N222L(S)
<400> 20
   ggtgtagagc tcatacttga g
   21
<210> 21
   <211> 20
   <212> DNA
   <213> Artificial Sequence
<220>
   <223> hIL-12(S)
<400> 21
   aactcgaggt cgacggtatc
   20
<210> 22
   <211> 19
   <212> DNA
   <213> Artificial Sequence
<220>
   <223> hIL-12(AS)
<400> 22
   ttctcgagcg gccgcacct
   19
<210> 23
   <211> 328
   <212> PRT
   <213> Artificial Sequence
<220>
   <223> human IL-12p40
<400> 23
   Met Cys His Gln Gln Leu Val Ile Ser Trp Phe Ser Leu Val Phe Leu
    1               5                  10                  15
   Ala Ser Pro Leu Val Ala Ile Trp Glu Leu Lys Lys Asp Val Tyr Val
               20                  25                  30
   Val Glu Leu Asp Trp Tyr Pro Asp Ala Pro Gly Glu Met Val Val Leu
           35                  40                  45
   Thr Cys Asp Thr Pro Glu Glu Asp Gly Ile Thr Trp Thr Leu Asp Gln
       50                  55                  60
   Ser Ser Glu Val Leu Gly Ser Gly Lys Thr Leu Thr Ile Gln Val Lys
   65                  70                  75                  80
   Glu Phe Gly Asp Ala Gly Gln Tyr Thr Cys His Lys Gly Gly Glu Val
                   85                  90                  95
   Leu Ser His Ser Leu Leu Leu Leu His Lys Lys Glu Asp Gly Ile Trp
               100                 105                 110
   Ser Thr Asp Ile Leu Lys Asp Gln Lys Glu Pro Lys Asn Lys Thr Phe
           115                 120                 125
   Leu Arg Cys Glu Ala Lys Asn Tyr Ser Gly Arg Phe Thr Cys Trp Trp
       130                 135                 140
   Leu Thr Thr Ile Ser Thr Asp Leu Thr Phe Ser Val Lys Ser Ser Arg
   145                 150                 155                 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
   195 200 205
   Glu Val Met Val Asp Ala Val His Lys Leu Lys Tyr Glu Asn Tyr Thr
   210 215 220
   Ser Ser Phe Phe Ile Arg Asp Ile Ile Lys Pro Asp Pro Pro Lys Asn
   225 230 235 240
   Leu Gln Leu Lys Pro Leu Lys Asn Ser Arg Gln Val Glu Val Ser Trp
   245 250 255
   Glu Tyr Pro Asp Thr Trp Ser Thr Pro His Ser Tyr Phe Ser Leu Thr
   260 265 270
   Phe Cys Val Gln Val Gln Gly Lys Ser Lys Arg Glu Lys Lys Asp Arg
   275 280 285
   Val Phe Thr Asp Lys Thr Ser Ala Thr Val Ile Cys Arg Lys Asn Ala
   290 295 300
   Ser Ile Ser Val Arg Ala Gln Asp Arg Tyr Tyr Ser Ser Ser Trp Ser
   305 310 315 320
   Glu Trp Ala Ser Val Pro Cys Ser
   325
<210> 24
   <211> 335
   <212> PRT
   <213> Artificial Sequence
<220>
   <223> mouse IL-12p40
<400> 24
   Met Cys Pro Gln Lys Leu Thr Ile Ser Trp Phe Ala Ile Val Leu Leu
   1 5 10 15
   Val Ser Pro Leu Met Ala Met Trp Glu Leu Glu Lys Asp Val Tyr Val
   20 25 30
   Val Glu Val Asp Trp Thr Pro Asp Ala Pro Gly Glu Thr Val Asn Leu
   35 40 45
   Thr Cys Asp Thr Pro Glu Glu Asp Asp Ile Thr Trp Thr Ser Asp Gln
   50 55 60
   Arg His Gly Val Ile Gly Ser Gly Lys Thr Leu Thr Ile Thr Val Lys
   65 70 75 80
   Glu Phe Leu Asp Ala Gly Gln Tyr Thr Cys His Lys Gly Gly Glu Thr
   85 90 95
   Leu Ser His Ser His Leu Leu Leu His Lys Lys Glu Asn Gly Ile Trp
   100 105 110
   Ser Thr Glu Ile Leu Lys Asn Phe Lys Asn Lys Thr Phe Leu Lys Cys
           115                 120                 125
   Glu Ala Pro Asn Tyr Ser Gly Arg Phe Thr Cys Ser Trp Leu Val Gln
       130                 135                 140
   Arg Asn Met Asp Leu Lys Phe Asn Ile Lys Ser Ser Ser Ser Ser Pro
   145                 150                 155                 160
   Asp Ser Arg Ala Val Thr Cys Gly Met Ala Ser Leu Ser Ala Glu Lys
                   165                 170                 175
   Val Thr Leu Asp Gln Arg Asp Tyr Glu Lys Tyr Ser Val Ser Cys Gln
               180                 185                 190
   Glu Asp Val Thr Cys Pro Thr Ala Glu Glu Thr Leu Pro Ile Glu Leu
           195                 200                 205
   Ala Leu Glu Ala Arg Gln Gln Asn Lys Tyr Glu Asn Tyr Ser Thr Ser
       210                 215                 220
   Phe Phe Ile Arg Asp Ile Ile Lys Pro Asp Pro Pro Lys Asn Leu Gln
   225                 230                 235                 240
   Met Lys Pro Leu Lys Asn Ser Gln Val Glu Val Ser Trp Glu Tyr Pro
                   245                 250                 255
   Asp Ser Trp Ser Thr Pro His Ser Tyr Phe Ser Leu Lys Phe Phe Val
               260                 265                 270
   Arg Ile Gln Arg Lys Lys Glu Lys Met Lys Glu Thr Glu Glu Gly Cys
           275                 280                 285
   Asn Gln Lys Gly Ala Phe Leu Val Glu Lys Thr Ser Thr Glu Val Gln
       290                 295                 300
   Cys Lys Gly Gly Asn Val Cys Val Gln Ala Gln Asp Arg Tyr Tyr Asn
   305                 310                 315                 320
   Ser Ser Cys Ser Lys Trp Ala Cys Val Pro Cys Arg Val Arg Ser
                   325                 330                 335